# EUROPÄISCHE PATENTSCHRIFT

(11) **EP 0 568 577 B1**
(45) Veröffentlichungstag und Bekanntmachung des Hinweises auf die Patenterteilung: **08.01.1997**
(21) Anmeldenummer: 92903405.6
(22) Anmeldetag: 23.01.1992
(51) Int. Cl.: A61K 31/195, A61K 9/20, A61K 9/16

(54) **PERORAL APPLIZIERBARE ARZNEIFORM ZUR BEHANDLUNG ZENTRALER DOPAMINMANGELZUSTÄNDE**
ORALLY ADMINISTERABLE DRUGS FOR THE TREATMENT OF CENTRAL DOPAMINE DEFICIENCY CONDITIONS
FORMULE DE MEDICAMENT A ADMINISTRATION ORALE POUR LE TRAITEMENT DES INSUFFISANCES EN DOPAMINE DU SYSTEME NERVEUX CENTRAL

(30) Priorität: 23.01.1991 DE 4101873
(43) Veröffentlichungstag der Anmeldung: 10.11.1993
(73) Patentinhaber: ISIS PHARMA GMBH, 08056 Zwickau (DE)
(72) Erfinder: WENZEL, Udo, D-4020 Halle (DE); WEBER, Günther, D-9580 Zwickau (DE); METZNER, Jürgen, D-4020 Halle (DE); DÄRR, Alfred, D-7010 Leipzig (DE); FREITAG, Sabine, D-9540 Zwickau (DE); FLÖTHER, Frank-Ulrich, CH-8730 Uznach (CH); ALBERT, Frank-Michael, D-9590 Zwickau (DE); HAASE, Margit, D-3400 Göttingen (DE); LEISTNER, Edith, D-7042 Leipzig (DE)
(86) Internationale Anmeldenummer: DE9200043
(87) Internationale Veröffentlichungsnummer: WO9212710

(56) Entgegenhaltungen:
- EP-A- 0 147 780
- EP-A- 0 253 490
- WO-A-83/00093
- US-A- 3 584 113
- CHEMICAL ABSTRACTS, vol. 882, no. 10, 10. März 1975, Columbus, Ohio, US; abstract no. 64498U; NOBORU M. ET AL: 'Increasing solubility of tyrosin or 3,4- dihydroxyphenylalanine', Seite 472, Spalte 2; & JP-A-49 04 454

## Beschreibung

### Anwendungsgebiet der Erfindung

Die Erfindung betrifft eine peroral applizierbare Arzneiform, die eine Kombination der Arzneistoffe Levodopa und Carbidopa in definierten Verhältnissen beinhaltet sowie ein Verfahren zu deren Herstellung. Die Erfindung ist in der pharmazeutischen Industrie anwendbar und dient zur Bereitstellung eines zur Therapie des Morbus Parkinson einsetzbaren Arzneimittels.

### Bekannter technischer Hintergrund

Schwere Störungen der Bewegungsautomatik - das sogenannte Parkinson-Syndrom - kommen mit zunehmendem Lebensalter gehäuft vor. Der zerebrale Mangel des für die extrapyramidale Motorik notwendigen Neurotransmitters Dopamin in den Basalganglien des Gehirns der Erkrankten, insbesondere im Corpus striatum, als Folge einer Nigrostrialis-Degeneration unbekannter Ätiologie, führt zu einem Ungleichgewicht zwischen den die Bewegungsabläufe vermittelnden dopaminergen und cholinergen Neurotransmittersystemen. Eine Substitution des fehlenden biogenen Amines läßt sich durch Zufuhr seiner die Blut-Hirn-Schranke penetrierenden Vorstufe Levodopa realisieren, welche in die dopaminergen Neuronen aufgenommen und zu Dopamin decarboxyliert wird (Birkmayer, Hornkiewicz, Wien. Klin. Wochenschrift 73 (1961), 787). Es ist bekannt, daß die periphere Dopadecarboxylierung von Levodopa durch die gleichzeitige perorale Applikation eines geeigneten Decarboxylasehemmers, wie z.B. Carbidopa (DE-PS-30 12 602, US-PS-3,769,424) oder Benserazid (DE-PS-32 35 093) weitgehend unterdrückt wird, woraus ein deutlicher Anstieg der Levodopa-Serumspiegel resultiert, der eine relevante Reduzierung der therapeutisch notwendigen Dosis und eine damit verbundene Verminderung von gastrointestinalen und kardiovaskulären Nebenwirkungen ermöglicht.

Bekannt ist die Herstellung von Levodopa und Carbidopa enthaltenden Kapseln, Filmtabletten und im Magensaft schwimmfähigen Tabletten (GB-PS-1,243,474, US-PS-4,424,235, BE-PS-894 376).
Weiterhin ist bekannt, daß internationale Standards bisher lediglich eine sehr schnelle in vitro-Liberation fordern (USP XXI). Bekannt sind außerdem Methoden zur Herstellung von Arzneimitteln, aus denen die Arzneistoffe Levodopa und Carbidopa langsam und gleichzeitig freigesetzt werden. Diese Arzneimittel können beispielsweise polymere Matrices (EP-A-0 253 490, EP-A-0 320 051), Pellets (DE-PS-38 41 955, EP-A-0 260 236, EP-A- 0 324 947) oder auch mehrschichtige Formlinge (EP-A-0 302 693, EP-A- 0 314 206) sein. Bekannt ist außerdem die Herstellung von Pulvermischungen spezifischer Partikelgröße, aus denen Arzneistoffe über einen Zeitraum von ca. 10 Stunden langsam und gleichmäßig freigegeben werden, durch Sprühtrocknung wäßriger Systeme, die neben einem wasserlöslichen Arzneistoff ein wasserlösliches Polymerharz enthalten, welches sich unter den gewählten Trocknungsbedingungen in ein unlösliches Harz umwandelt (US-PS-3,584,113). Allgemein ist bekannt, daß die Umwandlung eines Arzneistoffes bzw. eines Arzneistoffgemisches zur Arzneiform ein wesentliches Mittel zur Beeinflussung der Bioverfügbarkeit ist. Sie ist gleichzeitig eine Methode, um durch geeignete pharmazeutischtechnologische Hilfsstoffe sehr niedrige Arzneistoffdosen handhabbar zu machen bzw. um Arzneistoffinkompatibilitäten in Arzneistoffgemischen entgegenzuwirken oder die chemische Stabilität eines oder mehrerer Arzneistoffe zu gewährleisten. Weiterhin ist für die Herstellung von Tabletten eine Vielzahl von Verfahren bekannt, deren Ziel es ist, pulverförmige Arzneistoffe bzw. Mischungen aus pulverförmigen Arzneistoffen und pharmazeutischtechnologischen Hilfsstoffen unter technischen Bedingungen in Tabletten zu überführen. Es ist bekannt, daß durch Zugabe geeigneter pharmazeutisch-technologischer Hilfsstoffe zu Arzneistoffen oder Arzneistoff-Hilfsstoff-Mischungen die Eigenschaften der Kompositionen, wie Stabilität, elektrostatische Aufladbarkeit, Fließ- und Tablettierverhalten sowie ihre Bioverfügbarkeit, beeinflußt und verändert werden können. Ebenso ist die Abfüllung von arzneistoffhaltigen Pulvermischungen, Granulaten, Pellets u. ä. in Hartgelatinekapseln beschrieben. Weiterhin ist bekannt, daß durch die Zugabe von Polyvinylalkoholen eine signifikante Verzögerung der Arzneistoffliberation erzielt werden kann (DD-A-301 323; U. Meyer, Diss., Berlin 1977), und daß mit Polyvinylalkohol allgemein schnell zerfallende feste Tabletten herstellbar sind (W. Rietschel, "Die Tablette", 104, Aulendorf, 1966).

### Detaillierte Beschreibung der Erfindung

Die zum Teil erhebliche Variabilität des klinischen Bildes der Parkinsonkrankheit erfordert eine sensible, individuell einstellbare medikamentöse Therapie. Einerseits müssen dem Therapeuten Arzneiformen zur Verfügung stehen, die durch eine sofortige vollständige Liberation der Arzneistoffe Levodopa und Carbidopa im Tagesablauf eingetretene Transmitterdefizite ausgleichen und damit eine schnelle Besserung des Gesamtzustandes der Patienten erreichen können. Andererseits treten aber bei einer nicht geringen Anzahl der Patienten auch infolge der zügigen Wirkstofffreigabe einer Langzeittherapie mit nachfolgend hohen Levodopa-Plasmaspiegeln unerwünschte und unangenehme, das Befinden stark beeinträchtigende Nebenwirkungen auf.

Der Erfindung liegt die Aufgabe zugrunde, eine peroral applizierbare Arzneiform sowie ein Verfahren zu ihrer Herstellung zu entwickeln, nach dem aus Levodopa und Carbidopa Tabletten und Kapseln hergestellt werden können, aus denen die Arzneistoffe im Anflutungszeitraum von etwa 30 bis 45 min gesteuert und über einen weiteren gewünschten Zeitraum hinweg vollständig und gleichmäßig, aber nicht signifikant verzögert, freigegeben werden.

Erfindungsgemäß wird diese Aufgabe gelöst durch die in Patentanspruch 1 definierte, peroral applizierbare Arzneiform zur Behandlung zentraler Dopaminmangelzustände mit nach Bedarf gesteuerter Arzneistoffliberation in der Anflutungsphase, wobei die Arzneiform ein Gemisch, zusammengesetzt aus 100 bis 250 Masseteilen Levodopa, 10 bis 25 Masseteilen Carbidopa und 10 bis 200%, bezogen auf die Menge an Arzneistoffen, eines Polymerengemisches, bestehend aus Polyvinylalkoholen verschiedener Verseifungsgrade oder eines vollverseiften Polyvinylalkohols mit einem von Null verschiedenen Restacetylgehalt oder eines teilverseiften Polyvinylalkohols, enthält. Dieser Arzneistoff-Polymeren-Mischung werden übliche galenische Hilfsstoffe in einer Menge zugesetzt, welche es ermöglicht, die Arzneiform jeweils in an sich bekannter Weise herzustellen, so zum Beispiel durch Direkttablettierung, Tablettierung nach Granulierung mit einem geeigneten Bindemittel oder durch Abfüllung in Hartgelatinekapseln. Als Polyvinylalkohole werden vorzugsweise kommerzielle Produkte verwendet, deren k-Werte (Fikentscher, Cellulosechemie 13 (1932), 58) für vollverseifte Polyvinylalkohole in einem Bereich von 40 bis 59 und für teilverseifte Polyvinylalkohole in einem Bereich von 50 bis 59 liegen. Besonders geeignet sind vollverseifte Polyvinylalkohole mit einem von Null verschiedenen Restacetylgehalt bis 3 Prozent, einer mittleren Molmasse von 60000 bis 80000, einer Gesamtoberfläche von 0,1 m²/g bis 0,18 m²/g und teilverseifte Polyvinylalkohole mit einem Restacetylgehalt von 10 bis 18 Prozent, einer mittleren Molmasse von 80000, einer Gesamtoberfläche von 0,5 m²/g bis 0,69 m²/g und einem spezifischen Porenvolumen von 0,2 cm³/g bis 0,36 cm³/g.

Überraschenderweise wurde gefunden, daß die erfindungsgemäße Mischung der Hilfs- und Arzneistoffe und die dadurch mögliche einfache pharmazeutisch-technologische Verarbeitung zur Lösung der Aufgabenstellung führt. Weiterhin wurde überraschenderweise gefunden, daß man durch das erfindungsgemäße Verfahren, dem Einsatz von Kombinationen von Polyvinylalkoholen verschiedener Restacetylgehalte, Levodopa/Carbidopa-Arzneiformulierungen erhält, die die Arzneistoffe schnell und vollständig freigeben, aber deren Arzneistoffliberation in der Anflutungsphase nach Bedarf variiert bzw. gesteuert werden kann.

Das erfindungsgemäße Vorgehen führt zu einer Arzneiform, die beide Arzneistoffe schnell und über einen bestimmten Zeitraum hinweg in definierten Verhältnissen freisetzt und somit eine optimale Bioverfügbarkeit des peripheren Dopadecarboxylasehemmers Carbidopa und des Dopamin-Precursors Levodopa sichert. Für verschiedene Patientengruppen ist es dadurch möglich, mit minimalem Aufwand Arzneiformen mit jeweils optimalem Freigabeverhalten bereitzustellen. Mit der beschriebenen Arzneiform kann somit entsprechend dem klinischen Bild dem Erfordernis des Einsatzes von Levodopa/Carbidopa-Arzneiformen mit unverzüglicher und vollständiger bzw. initial verzögerter Liberation nachgekommen werden.

### Ausführungsbeispiele

### Beispiel 1 - 12

Levodopa wird mit Carbidopa, einem vollverseiften Polyvinylalkohol mit einem Restacetylgehalt von 2,5 %, einer mittleren Molmasse von 70000, einer Gesamtoberfläche von 0,14 m²/g und einem k-Wert von 55, im folgenden als PVA 1 bezeichnet, einem teilverseiften Polyvinylalkohol mit einem Restacetylgehalt von 15%, einer mittleren Molmasse von 80000, einer Gesamtoberfläche von 0,57 m²/g, einem spezifischen Porenvolumen von 0,3 cm³/g und einem mittleren k-Wert von 55, im folgenden als PVA 2 bezeichnet, und Magnesiumstearat gemischt und mit einer Preßkraft von 10 - 50 kN direkt zu Tabletten verpreßt.
Untersuchte Tablettenrezepturen: Tabelle 1
in vitro-Arzneistoffliberation: Tabelle 2

### Beispiel 13

250 g Levodopa, 25 g Carbidopa, 25 g eines vollverseiften Polyvinylalkoholes mit einem Restacetylgehalt von 3 %, einer mittleren Molmasse von 60000, einer Gesamtoberfläche von 0,104 m²/g und einem k-Wert von 45, 100 g Cellulosepulver mit einem Kornspektrum von < 0,16 mm ≥ 65 % und < 0,05 mm 10 bis 30 % und 5 g Magnesiumstearat werden gemischt und mit 100 ml einer 2%igen Gelatinelösung im Wirbelschichtgranulator bei 60 °C granuliert und bis zu einem Restwassergehalt von 3 bis 4 % getrocknet. Das Granulat wird durch anschließende Siebung auf eine maximale Korngröße von 1,2 mm gebracht und zu Tabletten mit einer Sollmasse von 407 mg bei einer Preßkraft von 10 - 50 kN verpreßt.
in vitro-Arzneistoffliberation: Tabelle 2

### Beispiel 14

250 g Levodopa, 25 g Carbidopa, 94 g Cellulosepulver mit einem Kornspektrum von < 0,16 mm ≥ 65 % und < 0,05 mm 10 bis 30 %, 3 g Siliziumdioxid und 51 g PVA 1 werden im Wirbelschichtgranulator gemischt, mit 10 ml einer 20%igen wäßrigen Zitronensäurelösung besprüht und mit 150 ml einer 5%igen wäßrigen Lösung von PVA 1 granuliert und gleichzeitig bei einer Temperatur von 60 °C bis zu einem Restwassergehalt von 3 bis 4 % getrocknet. Das Granulat wird durch Siebung auf eine maximale Korngröße von 1,2 mm gebracht, mit 30 g Talk und 5 g Magnesiumstearat 15 min gemischt und bei einer Preßkraft von 10 - 50 kN zu Tabletten mit einer Sollmasse von 440 mg verpreßt.
in vitro-Arzneistoffliberation: Tabelle 2

### Beispiel 15

100 g Levodopa, 25 g Carbidopa, 16 g PVA 1, 5 g PVA 2 und 3 g Siliziumdioxid werden im Wirbelschichtgranulator gemischt, mit 10 ml einer 20%igen Zitronensäurelösung besprüht und mit 75 ml einer 10%igen wäßrigen Lösung von PVA 1 granuliert und gleichzeitig bei einer Temperatur von 60 °C bis zu einem Restwassergehalt von 3 - 4 % getrocknet. Das Granulat wird durch Siebung auf eine maximale Korngröße von 1,00 mm gebracht, mit 90 g Cellulosepulver mit einem Kornspektrum von < 0,16 mm ≥ 65 % und < 0,05 mm 10 bis 30 %, 10 g Talk und 2 g Magnesiumstearat 15 min gemischt und jeweils zu 260 mg in eine Hartgelatinekapsel gefüllt.
in vitro-Arzneistoffliberation: Tabelle 2

**Tabelle 1**

| Beispiel | Levodopa [mg] | Carbidopa [mg] | PVA 1 [mg] | PVA 2 [mg] | Mg-Stearat [mg] |
|---|---|---|---|---|---|
| 1 | 100,0 | 10,0 | 100,0 | 10,0 | 5,0 |
| 2 | 100,0 | 25,0 | 100,0 | 25,0 | 5,0 |
| 3 | 150,0 | 15,0 | 150,0 | 15,0 | 5,0 |
| 4 | 250,0 | 25,0 | 250,0 | 25,0 | 5,0 |
| 5 | 250,0 | 25,0 | 25,0 | 100,0 | 5,0 |
| 6 | 250,0 | 25,0 | 125,0 | 0,0 | 5,0 |
| 7 | 250,0 | 25,0 | 0,0 | 125,0 | 5,0 |
| 8 | 250,0 | 25,0 | 100,0 | 25,0 | 5,0 |
| 9 | 100,0 | 25,0 | 2,5 | 10,0 | 5,0 |
| 10 | 100,0 | 25,0 | 12,5 | 50,0 | 5,0 |
| 11 | 100,0 | 25,0 | 25,0 | 100,0 | 5,0 |
| 12 | 100,0 | 25,0 | 50,0 | 200,0 | 5,0 |

**Tabelle 2**

| Beispiel | Levodopa [%] | | | | Carbidopa [%] | | | |
|---|---|---|---|---|---|---|---|---|
| | Zeit [min] | | | | Zeit [min] | | | |
| | 15 | 30 | 45 | 60 | 15 | 30 | 45 | 60 |
| 1 | 82,3 | 95,4 | 99,0 | 101,0 | 81,4 | 93,5 | 99,2 | 100,0 |
| 2 | 85,2 | 93,1 | 100,3 | - | 83,2 | 91,5 | 98,7 | - |
| 3 | 83,6 | 94,4 | 100,1 | - | 84,7 | 86,4 | 99,0 | - |
| 4 | 89,6 | 97,7 | 100,5 | - | 85,3 | 97,6 | 100,4 | - |
| 5 | 42,1 | 69,2 | 86,9 | 100,2 | 33,6 | 70,4 | 91,2 | 98,0 |
| 6 | 87,3 | 98,1 | 100,0 | - | 86,7 | 87,2 | 103,0 | - |
| 7 | 35,2 | 45,4 | 70,9 | 82,9 | 34,9 | 46,6 | 69,2 | 80,1 |
| 8 | 74,7 | 86,3 | 95,1 | 103,0 | 74,2 | 83,3 | 98,4 | - |
| 9 | 47,2 | 75,5 | 93,7 | 98,3 | 42,9 | 75,1 | 92,8 | 98,4 |
| 10 | 43,6 | 73,5 | 100,0 | - | 40,2 | 73,0 | 92,9 | 98,7 |
| 11 | 32,4 | 44,2 | 59,4 | 68,3 | 30,7 | 40,3 | 53,6 | 61,4 |
| 12 | 27,7 | 38,6 | 53,1 | 64,8 | 28,6 | 37,4 | 51,1 | 65,8 |
| 13 | 74,3 | 98,6 | 102,0 | - | 87,0 | 100,0 | - | - |
| 14 | 89,2 | 92,1 | 93,0 | 94,2 | 85,2 | 90,4 | 92,6 | 95,4 |
| 15 | 86,4 | 94,1 | 98,9 | 99,7 | 84,8 | 92,3 | 98,1 | 100,1 |

Bei den Beispielen 7, 11 und 12 wurde die Arzneistofffreigabe über 60 Minuten hinaus geprüft:

| Beispiel | Levodopa [%] | | Carbidopa [%] | |
|---|---|---|---|---|
| | Zeit [min.] | | Zeit [min.] | |
| | 90 | 120 | 90 | 120 |
| 7 | 103,0 | - | 100,0 | - |
| 11 | 85,6 | 99,1 | 86,3 | 102,2 |
| 12 | 86,4 | 100,0 | 84,5 | 98,6 |

## Patentansprüche

1. Peroral applizierbare Arzneiform zur Behandlung zentraler Dopaminmangelzustände mit nach Bedarf gesteuerter Arzneistoffliberation in der Anflutungsphase, dadurch gekennzeichnet, daß die Arzneiform ein Gemisch, zusammengesetzt aus
a) 100 bis 250 Masseteilen Levodopa,
b) 10 bis 25 Masseteilen Carbidopa und
c) 10 bis 200%, bezogen auf die Menge an Arzneistoffen,
i) eines Polymerengemisches,
bestehend aus Polyvinylalkoholen verschiedener Verseifungsgrade oder
ii) eines vollverseiften Polyvinylalkohols
mit einem von Null verschiedenen Restacetylgehalt oder
iii) eines teilverseiften Polyvinylalkohols, sowie
d) eine geeignete Menge üblicher galenischer Hilfsstoffe enthält.

2. Arzneiform nach Anspruch 1, dadurch gekennzeichnet, daß das Polymerengemisch aus vollverseiften Polyvinylalkoholen und teilverseiften Polyvinylalkoholen zusammengesetzt ist.

3. Arzneiform nach Anspruch 2, dadurch gekennzeichnet, daß das Polymerengemisch aus einem vollverseiften Polyvinylalkohol und einem teilverseiften Polyvinylalkohol zusammengesetzt ist.

4. Arzneiform nach Anspruch 1, dadurch gekennzeichnet, daß der vollverseifte Polyvinylalkohol mit einem von Null verschiedenen Restacetylgehalt
i) einen Restacetylgehalt bis 3 Prozent,
ii) eine mittlere Molmasse von 60000 bis 80000 und
iii) eine Gesamtoberfläche von 0,1 m²/g bis 0,18 m²/g besitzt.

5. Arzneiform nach Anspruch 1, dadurch gekennzeichnet, daß der teilverseifte Polyvinylalkohol
i) einen Restacetylgehalt von 10 bis 18 Prozent,
ii) eine mittlere Molmasse von 80000,
iii) eine Gesamtoberfläche von 0,5 m²/g bis 0,69 m²/g und
iv) ein spezifisches Porenvolumen von 0,2 cm³/g bis 0,36 cm³/g besitzt.

6. Arzneiform nach Anspruch 3, dadurch gekennzeichnet, daß der vollverseifte Polyvinylalkohol
i) einen Restacetylgehalt bis 3 Prozent,
ii) eine mittlere Molmasse von 60000 bis 80000 sowie
iii) eine Gesamtoberfläche von 0,1 m²/g bis 0,18 m²/g und der teilverseifte Polyvinylalkohol
iv) einen Restacetylgehalt von 10 bis 18 Prozent,
v) eine mittlere Molmasse von 80000,
vi) eine Gesamtoberfläche von 0,5 m²/g bis 0,69 m²/g sowie
vii) ein spezifisches Porenvolumen von 0,2 cm³/g bis 0,36 cm³/g besitzt.

7. Verfahren zur Herstellung einer Arzneiform nach Anspruch 1, dadurch gekennzeichnet, daß man ein Gemisch, zusammengesetzt aus
a) 100 bis 250 Masseteilen Levodopa,
b) 10 bis 25 Masseteilen Carbidopa und
c) 10 bis 200%, bezogen auf die Menge an Arzneistoffen,
i) eines Polymerengemisches,
bestehend aus Polyvinylalkoholen verschiedener Verseifungsgrade oder
ii) eines vollverseiften Polyvinylalkohols
mit einem von Null verschiedenen Restacetylgehalt oder
iii) eines teilverseiften Polyvinylalkohols, herstellt
sowie dieses Gemisch mit einer geeigneten Menge üblicher galenischer Hilfsstoffe in an sich bekannter Weise weiterverarbeitet.

8. Verfahren nach Anspruch 7, dadurch gekennzeichnet, daß das Polymerengemisch aus vollverseiften Polyvinylalkoholen und teilverseiften Polyvinylalkoholen zusammengesetzt ist.

9. Verfahren nach Anspruch 8, dadurch gekennzeichnet, daß das Polymerengemisch aus einem vollverseiften Polyvinylalkohol und einem teilverseiften Polyvinylalkohol zusammengesetzt ist.

10. Verfahren nach Anspruch 7, dadurch gekennzeichnet, daß der vollverseifte Polyvinylalkohol mit einem von Null verschiedenen Restacetylgehalt
i) einen Restacetylgehalt bis 3 Prozent,
ii) eine mittlere Molmasse von 60000 bis 80000 und
iii) eine Gesamtoberfläche von 0,1 m²/g bis 0,18 m²/g besitzt.

11. Verfahren nach Anspruch 7, dadurch gekennzeichnet, daß der teilverseifte Polyvinylalkohol
i) einen Restacetylgehalt von 10 bis 18 Prozent,
ii) eine mittlere Molmasse von 80000,
iii) eine Gesamtoberfläche von 0,5 m²/g bis 0,69 m²/g und
iv) ein spezifisches Porenvolumen von 0,2 cm³/g bis 0,36 cm³/g besitzt.

12. Verfahren nach Anspruch 9, dadurch gekennzeichnet, daß der vollverseifte Polyvinylalkohol
i) einen Restacetylgehalt bis 3 Prozent,
ii) eine mittlere Molmasse von 60000 bis 80000 sowie
iii) eine Gesamtoberfläche von 0,1 m²/g bis 0,18 m²/g und der teilverseifte Polyvinylalkohol
iv) einen Restacetylgehalt von 10 bis 18 Prozent,
v) eine mittlere Molmasse von 80000,
vi) eine Gesamtoberfläche von 0,5 m²/g bis 0,69 m²/g sowie
vii) ein spezifisches Porenvolumen von 0,2 cm³/g bis 0,36 cm³/g besitzt.

## Claims

1. Pharmaceutical formulation which can be administered orally for the treatment of central dopamine deficiency conditions wherein the pharmaceutical substances are released in the flooding phase in a controlled manner as required, characterised in that the pharmaceutical formulation is a mixture composed of:
a) 100 to 250 parts by weight levodopa,
b) 10 to 25 parts by weight carbidopa and
c) 10 to 200%, in relation to the quantity of pharmaceutical substances,
i) of a polymer mixture
consisting of differently saponified polyvinyl alcohols or
ii) of a fully saponified polyvinyl alcohol
having a residual acetyl content other than zero or
iii) a partly saponified polyvinyl alcohol and
d) contains a suitable quantity of conventional galenical adjuvants.

2. Pharmaceutical formulation according to claim 1, characterised in that the polymer mixture is composed of fully saponified polyvinyl alcohols and partly saponified polyvinyl alcohols.

3. Pharmaceutical formulation according to claim 2, characterised in that the polymer mixture is composed of a fully saponified polyvinyl alcohol and a partly saponified polyvinyl alcohol.

4. Pharmaceutical formulation according to claim 1, characterised in that the fully saponified polyvinyl alcohol having a residual acetyl content other than zero comprises:
i) a residual acetyl content up to 3%
ii) an average molecular mass of 60000 to 80000 and
iii) a total surface of 0.1 m²/g to 0.18 m²/g.

5. Pharmaceutical formulation according to claim 1, characterised in that the partly saponified polyvinyl alcohol comprises:
i) a residual acetyl content of 10 to 18%
ii) an average molecular mass of 80000
iii) a total surface of 0.5 m²/g to 0.69 m²/g and
iv) a specific pore volume of 0.2 cm³/g to 0.36 cm³/g.

6. Pharmaceutical formulation according to claim 3, characterised in that the fully saponified polyvinyl alcohol comprises:
i) a residual acetyl content up to 3%,
ii) an average molecular mass of 60000 to 80000 and
iii) a total surface of 0.1 m²/g to 0.18 m²/g and the partly saponified polyvinyl alcohol comprises:
iv) a residual acetyl content of 10 to 18%,
v) an average molecular mass of 80000,
vi) a total surface of 0.5 m²/g to 0.69 m²/g and
vii) a specific pore volume of 0.2 cm³/g to 0.36 cm³/g.

7. Method of producing a pharmaceutical formulation according to claim 1, characterised in that a mixture is produced composed of:
a) 100 to 250 parts by weight levodopa,
b) 10 to 25 parts by weight carbidopa and
c) 10 to 200%, in relation to the quantity of pharmaceutical substances,
i) a polymer mixture
consisting of differently saponified polyvinyl alcohols or
ii) a fully saponified polyvinyl alcohol
having a residual acetyl content other than zero or
iii) a partly saponified polyvinyl alcohol
and this mixture is processed further in a manner known per se with a suitable quantity of conventional galenical adjuvants.

8. Method according to claim 7, characterised in that the polymer mixture is composed of fully saponified polyvinyl alcohols and partly saponified polyvinyl alcohols.

9. Method according to claim 8, characterised in that the polymer mixture is composed of a fully saponified polyvinyl alcohol and partly saponified polyvinyl alcohol.

10. Method according to claim 7, characterised in that the fully saponified polyvinyl alcohol having a residual acetyl content other than zero comprises:
i) a residual acetyl content up to 3%,
ii) an average molecular mass of 60000 to 80000 and
iii) a total surface of 0.1 m²/g to 0.18 m²/g.

11. Method according to claim 7, characterised in that the partly saponified polyvinyl alcohol comprises:
i) a residual acetyl content of 10 to 18%,
ii) an average molecular mass of 80000,
iii) a total surface of 0.5 m²/g to 0.69 m²/g and
iv) a specific pore volume of 0.2 cm³/g to 0.36 cm³/g.

12. Method according to claim 9, characterised in that the fully saponified polyvinyl alcohol comprises:
i) a residual acetyl content up to 3%,
ii) an average molecular mass of 60000 to 80000 and
iii) a total surface of 0.1 m²/g to 0.18 m²/g and the partly saponified polyvinyl alcohol comprises:
iv) a residual acetyl content of 10 to 18%,
v) an average molecular mass of 80000,
vi) a total surface of 0.5 m²/g to 0.69 m²/g and
vii) a specific pore volume of 0.2 cm³/g to 0.36 cm³/g.

## Revendications

1. Forme de médicament administrable par la voie perorale, destinée au traitement d'états de déplétion en dopamine avec libération de la substance médicamenteuse réglée ou dirigée selon les besoins au cours de la phase initiale, caractérisée en ce que la forme de médicament contient un mélange qui se compose de
a) 100 à 250 parties massiques de Lévodopa,
b) 10 à 25 parties massiques de Carbidopa et
c) 10 à 200%, par rapport à la quantité de substances médicamenteuses,
i) d'un mélange de polymères,
se composant de poly(alcools vinyliques) de degrés de saponification différents ou,
ii) d'un poly(alcool vinylique) totalement saponifié
avec une teneur en acétyle résiduelle qui diffère de zéro, ou
iii) d'un poly(alcool vinylique) partiellement saponifié, ainsi que
d) d'une proportion appropriée d'adjuvants ou excipients galéniques habituels.

2. Forme de médicament suivant la revendication 1, caractérisée en ce que le mélange de polymères est constitué de poly(alcools vinyliques) totalement saponifiées et de poly(alcools vinyliques) partiellement saponifiés.

3. Forme de médicament suivant la revendication 2, caractérisée en ce que que le mélange de polymères est constitués d'un poly(alcool vinylique) totalement saponifiée et d'un poly(alcool vinylique) partiellement saponifié.

4. Forme de médicament suivant la revendication 1, caractérisée en ce que le poly(alcool vinylique) totalement saponifié avec une teneur en acétyle résiduelle qui diffère de zéro, possède
i) une teneur en acétyle résiduelle allant jusqu'à 3%,
ii) une masse moléculaire moyenne de 60000 à 80000 et
iii) une surface totale de 0,1 m²/g à 0,18 m²/g.

5. Forme de médicament suivant la revendication 1, caractérisée en ce que le poly(alcool vinylique) partiellement saponifié possède
i) une teneur en acétyle résiduelle 10 à 18%,
ii) une masse moléculaire moyenne de 80000,
iii) une surface totale de 0,5 m²/g à 0,69 m²/g et
iv) un volume des pores spécifique de 0,2 cm³/g à 0,36 cm³/g.

6. Forme de médicament suivant la revendication 3, caractérisée en ce que le poly(alcool vinylique) totalement saponifié possède
i) une teneur en acétyle résiduelle allant jusqu'à 3%,
ii) une masse moléculaire moyenne de 60000 à 80000, ainsi que
iii) une surface totale de 0,1 m²/g à 0,18 m²/g le poly(alcool vinylique) partiellement saponifié possède
iv) une teneur en acétyle résiduelle allant 10 à 18%,
v) une masse moléculaire moyenne de 80000,
vi) une surface totale de 0,5 m²/g à 0,69 m²/g ainsi que
vii) un volume des pores spécifique de 0,2 cm³/g à 0,36 cm³/g.

7. Procédé de préparation d'une forme de médicament suivant la revendication 1, caractérisé en ce que l'on prépare un mélange constitué de
a) 100 à 250 parties massiques de Lévodopa,
b) 10 à 25 parties massiques de Carbidopa et
c) 10 à 200%, par rapport à la masse des substances médicamenteuses,
i) d'un mélange de polymères
constitué de poly(alcools vinyliques) de degrés de saponification différents ou
ii) d'un poly(alcool vinylique) totalement saponifié
avec une teneur en acétyle résiduelle qui diffère de zéro, ou
iii) d'un poly(alcool vinylique) partiellement saponifié,
et on poursuit le traitement de ce mélange avec une proportion appropriée d'adjuvants ou d'excipients galéniques habituels d'une manière en soi connue.

8. Procédé suivant la revendication 7, caractérisé en ce que le mélange de polyméres se compose de poly(alcools vinyliques) totalement saponifiés et de poly(alcools vinyliques) partiellement saponifiés

9. Procédé suivant la revendication 8, caractérisé en ce que le mélange de polyméres se compose d'un poly(alcool vinylique) totalement saponifié et d'un poly(alcool vinylique) partiellement saponifié.

10. Procédé suivant la revendication 7, caractérisé en ce que le poly(alcool vinylique) totalement saponifié avec une teneur en acétyle résiduelle qui diffère de zéro, possède
i) une teneur en acétyle résiduelle allantjusqu'à 3%,
ii) une masse moléculaire moyenne de 60000 à 80000 et
iii) une surface totale de 0,1 m²/g à 0,18 m²/g.

11. Procédé suivant la revendication 7, caractérisé en ce que le poly(alcool vinylique) partiellement saponifié possède
i) une teneur en acétyle résiduelle 10 à 18%,
ii) une masse moléculaire moyenne de 80000,
iii) une surface totale de 0,5 m²/g à 0,69 m²/g et
iv) un volume des pores spécifique de 0,2 cm³/g à 0,36 cm³/g.

12. Procédé suivant la revendication 9, caractérisé en ce que le poly(alcool vinylique) totalement saponifié possède
i) une teneur en acétyle résiduelle allant jusqu'à 3%,
ii) une masse moléculaire moyenne de 60000 à 80000, ainsi que
iii) une surface totale de 0,1 m²/g à 0,18 m²/g le poly(alcool vinylique) partiellement saponifié possède
iv) une teneur en acétyle résiduelle allant de 10 à 18%,
v) une masse moléculaire moyenne de 80000,
vi) une surface totale de 0,5 m²/g à 0,69 m²/g ainsi que
vii) un volume des pores spécifique de 0,2 cm³/g à 0,36 cm³/g.
